# EUROPÄISCHE PATENTSCHRIFT

(11) **EP 1 234 567 B1**
(45) Veröffentlichungstag und Bekanntmachung des Hinweises auf die Patenterteilung: **11.04.2007**
(21) Anmeldenummer: 02003250.4
(22) Anmeldetag: 21.02.2002
(51) Int. Cl.: A61K 6/083

(54) **Füllstoff auf der Basis von partikulärem Komposit**
Filling material based on particulate composite
Matériau de remplissage à base d'un composite particulaire

(30) Priorität: 21.02.2001 DE 10108261
(43) Veröffentlichungstag der Anmeldung: 28.08.2002
(73) Patentinhaber: Ivoclar Vivadent AG, 9494 Schaan (LI)
(72) Erfinder: Vogel, Karin, 9487 Gamprin (LI); Neubert, Roland, 6800 Feldkirch (AT); Salz, Ulrich, 88131 Lindau (DE); Rheinberger, Volker, 9490 Vaduz (LI)
(74) Vertreter: UEXKÜLL & STOLBERG

(56) Entgegenhaltungen:
- EP-A- 0 394 798
- EP-A- 0 475 239
- WO-A-00/61073
- WO-A-99/65453
- DE-A- 2 705 220
- DE-A- 3 502 594
- DE-A- 19 918 974
- US-A- 5 356 951

## Beschreibung

Die Erfindung betrifft Zusammensetzungen, die Füllstoffe auf der Basis von partikelförmigen Kompositwerkstoffen mit definierter Korngrößenverteilung und nur einem geringen Anteil feinkörniger Partikel enthalten.

Moderne Dentalwerkstoffe enthalten in der Regel als eine wesentliche Komponente ein flüssiges, polymerisierbares Bindemittel in Form von Monomeren oder Monomermischungen. Es ist bekannt, daß bei der Polymerisation solcher Bindemittel gewöhnlich eine mehr oder weniger stark ausgeprägte Volumenkontraktion stattfindet (vgl. R.R. Sadhir, R.M. Luck, Expanding Monomers - Synthesis, Characterization and Application; CRC Press, Boca Raton 1992, Seiten 3ff). Das Schrumpfen ist auf die Ausbildung kovalenter Bindungen zwischen den Monomermolekülen bei der Polymerisation zurückzuführen, wodurch der Abstand zwischen den Molekülen verringert wird. Im unpolymerisierten Zustand werden die Moleküle überwiegend durch Van der Waals-Kräfte zusammengehalten, welche einen größeren intermolekularen Abstand zur Folge haben.

Bei der Herstellung von Formteilen wirkt sich der Polymerisationsschrumpf sehr nachteilig auf die Dimensionsstabilität und die mechanischen Eigenschaften der Formkörper aus. Bei Adhäsiven und Klebeverbindungen beeinträchtigt der Polymerisationsschrumpf die Haftungseigenschaften und die Verbundfestigkeit, was bei Dentalmaterialien die Haftung zwischen Restaurationsmaterial und der natürlichen Zahnsubstanz verschlechtert. Es kommt zur Ausbildung von Spalten, welche die Bildung von Sekundärkaries begünstigen.

Zur Verringerung des Polymerisationsschrumpfes von Dentalmaterialien wurde der Einsatz von hochmolekularen Monomeren oder Präpolymeren, die Verwendung von Monomeren, die sich durch Ringöffnungspolymerisation polymerisieren lassen, die Zugabe von inerten, porösen oder expandierenden Füllstoffen oder Gas freisetzenden Systemen beschrieben.

Bei der Auswahl geeigneter Füllmaterialien kommt der Partikelgröße eine besondere Bedeutung zu. Große Partikeldurchmesser ergeben Materialien mit schlechter Polierbarkeit und inakzeptabler Abrasion, während feinteilige Füllmaterialien eine starke Verdickungswirkung zeigen, wodurch der Monomerbedarf erhöht und als Folge davon der Polymerisationsschrumpf vergrößert wird. Außerdem wird durch die hohe Viskosität das Einarbeiten des Füllstoffs erschwert und die maximale Füllstoffmenge begrenzt.

Aus der DE-OS 14 92 040 sind Zahnfüllmassen bekannt, die als Füllstoff Glasperlen mit einer Teilchengröße im Bereich von 5 bis 100 µm enthalten. Als Zwischenraumfüllstoff werden zusätzlich Glasfasern eingesetzt. Die Perlen sollen auf Grund ihrer Kugelform eine optimale Bruchfestigkeit und eine geringe Abriebswirkung gewährleisten. Die Farbe der Massen soll sich beim Aushärten von selbst an die Farbe des natürlichen Zahnmaterials anpassen.

Die DE 24 03 211 A1 offenbart Dentalwerkstoffe, in denen ausschließlich mikrofeine anorganische Füllstoffe (Mikrofüllstoffe) auf Siliciumdioxidbasis eingesetzt werden, deren Teilchengröße unter 700 nm liegt. Gemäß einer besonders bevorzugten Ausführungsform haben mindestens 50 Gew.-% des Füllstoffs eine Teilchengröße von 10 bis 400 nm. Man erhält überraschend Füllungsmaterialien mit guter Transparenz und Polierbarkeit sowie ausgezeichneten physikalischen Eigenschaften.

Die DE 27 05 220 A1 schlägt transparente Dentalwerkstoffe mit hoher Druckfestigkeit vor, in denen ein feinteiliger Füllstoff mit einer solchen Kornverteilung eingesetzt wird, daß 70 - 95 % der Teilchen eine Korngröße von 0,7 bis 25 µm und 5 - 30 % der Teilchen eine Korngröße von 0,2 bis 0,7 µm aufweisen. Darüber hinaus kann der Füllstoff in einer Menge bis zu 5 Gew.-% Teilchen mit einem geringeren Durchmesser als 0,2 µm enthalten. Die mittlere Korngröße der feinteiligen Füllstoffe wird mit 1 - 5 µm angegeben. Nach den Beispielen wird roher α-Quarz erhitzt und nach einer bestimmten Methode aufgemahlen.

Die EP 0 475 239 A2 offenbart Dentalmaterialien, die als Füllstoff eine Mischung aus amorphen, kugelförmigen Teilchen aus Siliciumdioxid und bis zu 20 Mol-% eines Oxids mindestens eines Elements der Gruppen I, II, III und IV des Periodensystems mit einem Brechungsindex von 1,50 bis 1,58 und mit einer durchschnittlichen Primärteilchengröße von 0,1 bis 1,0 µm, und Quarz-, Glaskeramik- oder Glaspulver oder deren Mischungen mit einem Brechungsindex von 1,50 bis 1,58 und mit einer durchschnittlichen Teilchengröße von 0,5 bis 5,0 µm enthalten. Die Materialien zeichnen sich durch eine hohe Transparenz und gute Polierbarkeit aus.

Die US 5,356,951 offenbart Dentalwerkstoffe, die als Füllstoff eine Mischung aus einem organisch-anorganischen Kompositfüller mit einer mittleren Partikelgröße von 5 bis 50 µm, Glaspulver mit einer maximalen Partikelgröße von 10 µm und einer mittleren Partikelgröße von 0, 1 bis 5 µm und einem feinteiligen Füllstoff mit einer Partikelgröße von 0,01 bis 0,04 µm enthalten. Der Kompositfüller ist seinerseits mit Glaspulver gefüllt, das eine maximale Teilchengröße von 10 µm und eine mittlere Teilchengröße von 0,1 bis 5 µm aufweist. Die Werkstoffe sollen sich durch eine glatte Oberfläche, einen geringen Polymerisationsschrumpf und verbesserte physikalische Eigenschaften auszeichnen.

Die DE 198 23 530 A1 offenbart Dentalmaterialien, die als Füllstoff ein organisch-anorganisches Verbundmaterial enthalten können, das eine mittlere Teilchengröße von 5 bis 50 µm aufweist und seinerseits mit einem ultrafeinen anorganischen Füllstoff mit einer mittleren Teilchengröße von 0,01 bis 0,04 µm gefüllt ist. Die Dentalmaterialien werden unter Anwendung von Druck und Hitze polymerisiert und dann durch Fräsen zu Dentalrestaurationen weiterverarbeitet. Diese sollen sich durch gute mechanische Eigenschaften auszeichnen und frei von unpolymerisiertem Monomer sein.

Die EP 0 983 762 A1 offenbart Dentalmaterialien, die als Füllstoff eine Mischung aus organisch-anorganischem Kompositfüller mit einer mittleren Partikelgröße von 5 bis 50 µm, partikulärem Füllstoff mit einer mittleren Partikelgröße von 20 nm oder weniger und ggf. Glaspulver mit einer maximalen Partikelgröße von 5 µm und einer mittleren Partikelgröße von 0,5 bis 2 µm enthalten. Der Kompositfüller wird durch Härten einer Mischung aus einem partikulären Füllstoff mit einer mittleren Partikelgröße von 20 nm oder weniger und einem Methacrylat- oder Acrylatmonomer mit einer Viskosität von 60 cP oder mehr und Pulverisieren der gehärteten Mischung hergestellt. Die Materialien sollen sich durch gute Polierbarkeit und gute mechanische Eigenschaften auszeichnen und eine dem natürlichen Zahn entsprechende Glätte und Transparenz aufweisen.

Trotz der zahlreichen im Stand der Technik beschriebenen Dentalwerkstoffe und trotz der zum Teil beachtlichen Verbesserungen, die hinsichtlich bestimmter Materialeigenschaften erzielt wurden, weisen herkömmliche Dentalwerkstoffe meist immer noch einen Polymerisationsschrumpf von 2,3 bis 2,8 % auf. Es besteht daher nach wie vor ein Bedürfnis, den Polymerisationsschrumpf von Dentalmaterialien ohne Beeinträchtigung der übrigen Eigenschaften weiter zu reduzieren.

Der Erfindung liegt demnach die Aufgabe zugrunde, polymerisierbare Zusammensetzungen bereitzustellen, die einen geringen Polymerisationsschrumpf und gute sonstige Eigenschaften wie Transparenz und Polierbarkeit aufweisen.

Diese Aufgabe wird durch polymerisierbare Zusammensetzungen gelöst, die mindestens ein polymerisierbares Monomer und/oder Präpolymer, mindestens einen Polymerisationsinitiator und 20 bis 90 Gew.-%, bezogen auf die Gesamtmasse der Zusammensetzung, mindestens eines neuartigen Füllstoffs auf der Basis von partikulärem Kompositmaterial enthalten. Dieser Kompositfüller weist eine mittlere Partikelgröße von 20 bis 50 µm auf und enthält maximal 10 Gew.-% Partikel mit einer Korngröße von < 10 µm. Die Prozentangabe bezieht sich auf die Masse des Kompositfüllers. Unter einem Kompositmaterial wird ein Material auf der Basis von polymerisierbarem organischem Bindemittel und anorganischem Füllstoff verstanden. Die Zusammensetzung ist im wesentlichen frei von Füllstoff mit einer Partikelgrösse von < 100mm.

Es wurde überraschend gefunden, daß durch die Verwendung von Kompositfüller mit der angegebenen Korngrößenverteilung der Polymerisationsschrumpf polymerisierbarer Zusammensetzungen deutlich verringert werden kann. Zudem zeichnen sich die Zusammensetzungen nach dem Härten trotz ihres vergleichsweise hohen Gehalts an grobkörnigem Füllstoff durch gute Polierbarkeit, Oberflächenglätte und Abrasion aus.

Grundsätzlich sind Kompositfüller bevorzugt, die einen möglichst geringen Anteil an feinpartikulärem Material enthalten, insbesondere maximal 8 Gew.-% und besonders bevorzugt maximal 6 Gew.-% Partikel mit einer Größe von < 10 µm. Weiter bevorzugt sind Kompositfüller mit einer mittleren Partikelgröße von 30 bis 40 µm. Die maximale Partikelgröße der Kompositfüller beträgt vorzugsweise 70 µm, d.h. das Material enthält weniger als 5 Gew.-%, besonders bevorzugt weniger als 1 Gew.-% Partikel mit einer Größe von mehr als 70 µm.

Unter der mittleren Partikelgröße wird, wenn nicht anders angegeben, das Zahlenmittel verstanden. Dieses ergibt sich aus der Häufigkeitsverteilung nach dem Klassieren.

Erfindungsgemäß als Kompositfüller geeignete partikuläre Kompositmaterialien lassen sich beispielsweise durch Mischen von organischem Bindemittel, Füllstoff und gegebenenfalls Polymerisationsinitiator, Härten und anschließendes Mahlen der Mischung herstellen. Das Mahlgut wird, falls erforderlich, klassiert, um Füllstoff mit der gewünschten Korngrößenverteilung zu erhalten.

Das Zerkleinern kann in herkömmlichen Mühlen erfolgen, beispielsweise in einer Kugelmühle, Luftstrahlmühle, Prallmühle oder Vibrationsmühle. Das Kompositmaterial kann vor dem eigentlichen Mahlen zum Beispiel in einem Kegelbrecher vorgebrochen werden.

Die Ausführungsbeispiele zeigen, daß beim gewöhnlichen Mahlen von Kompositmaterialien ein erheblicher Anteil an feinpartikulärem Material mit einem Partikeldurchmesser von < 10 µm gebildet wird. Dieser wird erfindungsgemäß abgetrennt, beispielsweise durch Klassieren, wie Stromklassieren, Sichten, Windsichten, gegebenenfalls in Kombination mit elektrostatischen Verfahren, Flotation, Sedimentation, elektrostatische oder magnetische Trennung oder Sieben. Geeignete Verfahren werden in Ullmanns Encyklopädie der Technischen Chemie, Band 2 (1988), Unit Operations, beschrieben.

Als organisches Bindemittel zur Herstellung der partikulären Komposite eignen sich alle durch Polymerisation härtbare Bindemittel, insbesondere ethylenisch ungesättigte, polymerisierbare Bindemittel, z.B. Monomere, wie monofunktionelle oder polyfunktionelle Methacrylate, die allein oder in Mischungen eingesetzt werden können. Als bevorzugte Beispiele für diese Verbindungen kommen Methylmethacrylat, Isobutylmethacrylat, Cyclohexylmethacrylat, Tetraethylenglycoldimethacrylat, Triethylenglycoldimethacrylat, Diethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-[4-(2-Hydroxy-3-methacryloxypropoxy)-phenyl]-propan (Bis-GMA) sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tri(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat, die im folgenden als Urethandimethacrylate bezeichnet werden. Der Bindemittelanteil bewegt sich zwischen 10 und 80 Gew.-% bezogen auf die Masse des Kompositmaterials, vorzugsweise 10 bis 30 Gew.-%.

Besonders bevorzugte Monomere sind Urethandimethacrylat (UDMA), d.h. das Umsetzungsprodukt von 1 Mol 2,2,4-Trimethylhexamethylendiisocyanat mit 2 Mol 2-Hydroxyethylmethacrylat, 1,10-Decandioldi(meth)acrylat, Bisphenol-A-Dimethacrylat, ethoxyliertes Bisphenol-A-Dimethacrylat und Mischungen dieser Monomere.

Zur Initiierung der Polymerisation enthalten die Mischungen einen Polymerisationsinitiator, beispielsweise einen Initiator für die radikalische Polymerisation. Je nach Art des verwendeten Initiators können die Mischungen kalt, durch Licht oder vorzugsweise heiß polymerisierbar sein.

Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet.

Als Initiatoren für die Photopolymerisation können z.B. Benzophenon und seine Derivate sowie Benzoin und seine Derivate verwendet werden. Weitere bevorzugte Photoinitiatoren sind die α-Diketone, wie 9,10-Phenanthrenchinon, Diacetyl, Furil, Anisil, 4,4'-Dichlorbenzil und 4,4'-Dialkoxybenzil. Campherchinon wird besonders bevorzugt verwendet.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Heißhärtung eingesetzt werden.

Photoinitiatoren werden vorzugsweise zusammen mit einem Reduktionsmittel verwendet. Beispiele für Reduktionsmittel sind Amine wie Cyanethylmethylanilin, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Initiatoren für die Kaltpolymerisation werden Radikale liefernde Systeme, z.B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Katalyse verwendet werden, z.B. Photoinitiatoren mit Aminen und Peroxiden.

Die Initiatoren werden üblicherweise in einer Menge von 0,01 bis 5 Gew.-% bezogen auf die Gesamtmasse der Mischung verwendet.

Als anorganischer Füllstoff eignen sich besonders Quarz-, Glaskeramik-, Glaspulver oder Mischungen davon, vorzugsweise Glaspulver und ganz besonders bevorzugt Bariumglaspulver und/oder Strontiumglaspulver, wobei die mittlere Partikelgröße dieser Pulver vorzugsweise im Bereich von 0,4 bis 1,5 µm und insbesondere im Bereich von 0,7 bis 1,0 µm liegt. Quarz-, Glaskeramik- und/oder Glaspulver werden vorzugsweise in einer Menge von 10 bis 80 Gew.-%, insbesondere 40 bis 65 Gew.-% bezogen auf die Gesamtmasse der Mischung verwendet.

Darüber hinaus kann der Kompositfüller Füllstoffe zur Erzielung einer erhöhten Röntgenopazität enthalten. Die mittlere Partikelgröße des röntgenopaken Füllstoffs liegt vorzugsweise im Bereich von 100 bis 300 nm, insbesondere 180 bis 300 nm. Als röntgenopake Füllstoffe eignen sich z.B. die in der DE 35 02 594 A1 beschriebenen Fluoride der Seltenen Erdmetalle, d.h. die Trifluoride der Elemente 57 bis 71. Ein besonders bevorzugt verwendeter Füllstoff ist Ytterbiumfluorid, insbesondere Ytterbiumtrifluorid mit einer mittleren Partikelgröße von etwa 300 nm. Die Menge des röntgenopaken Füllstoffs beträgt vorzugsweise 10 bis 50 Gew.-%, besonders bevorzugt 20 bis 30 Gew.-%.

Außerdem können gefällte Mischoxide, wie beispielsweise ZrO₂/SiO₂, als Füllstoffe eingesetzt werden. Bevorzugt sind Mischoxide mit einer Partikelgröße von 200 bis 300 nm und insbesondere etwa 200 nm. Die Mischoxidpartikel sind vorzugsweise kugelförmig und weisen eine einheitliche Größe auf. Die Mischoxide haben vorzugsweise einen Brechungsindex von 1,52 bis 1,55. Die Mischoxide können als alleiniger Füllstoff oder in Kombination mit anderen Füllern eingesetzt werden. Das gefällte Mischoxid wird vorzugsweise in einer Menge von 20 bis 90 Gew.-%, besonders bevorzugt 25 bis 75 Gew.-% und ganz besonders bevorzugt 40 bis 75 Gew.-% verwendet.

Die Füllstoffe sind zur Verbesserung der Haftung zwischen Füllstoff und organischer Matrix bevorzugt silanisiert. Als Haftvermittler eignet sich besonders α-Methacryloxypropyltrimethoxysilan. Die Menge des eingesetzten Haftvermittlers richtet sich nach der Art und BET-Oberfläche des Füllstoffs.

Zusätzlich zu den bereits genannten Stoffen können die Mischungen Additive, wie Stabilisatoren und Polymerisationsinhibitoren enthalten. Diese werden bevorzugt in einer Menge von 0,01 bis 2 Gew.-% verwendet.

Die Gesamtmenge an anorganischem Füllstoff liegt vorzugsweise im Bereich von 60 bis 88 Gew.-% bezogen auf die Gesamtmasse des Kompositfüllers.

Eine bevorzugte Mischung zur Herstellung des Kompositfüllers weist demnach folgende Zusammensetzung auf:
(a) 10 bis 80 Gew.-%, vorzugsweise 10 bis 30 Gew.-% organisches Bindemittel;
(b) 0,01 bis 5 Gew.-%, vorzugsweise 0,03 bis 5 Gew.-% und insbesondere 0,5 bis 2 Gew.-% Polymerisationsinitiator;
(c) 20 bis 90 Gew.-%, vorzugsweise 60 bis 88 Gew.-% anorganischer Füllstoff.

Als Füllstoff (c) enthält die Mischung vorzugsweise
(c1) ggf. 10 bis 80 Gew.-%, vorzugsweise 40 bis 65 Gew.-% Glaspulver; und/oder
(c2) ggf. 10 bis 50 Gew.-%, vorzugsweise 20 bis 30 Gew.-% röntgenopaken Füllstoff; und/oder
(c3) ggf. 20 bis 90 Gew.-%, vorzugsweise 25 bis 75 Gew.-%, besonders bevorzugt 40 bis 75 Gew.-% gefälltes Mischoxid.

Alle Angaben beziehen sich jeweils auf die Gesamtmasse der Mischung. Die Zusammensetzung kann eine der Komponenten (c1), (c2) oder (c3) oder eine Mischung daraus als Füller enthalten. Bevorzugt sind Zusammensetzungen, die einen Füllstoff des Typs (c1) enthalten, allein oder besonders bevorzugt im Kombination mit einer der Komponenten (c2) bis (c3).

Nach dem Härten, Mahlen und Klassieren werden die Kompositpartikel vorzugsweise mit einem geeigneten Haftvermittler behandelt. Dieser reagiert mit den freien Oberflächen des Füllstoffs der Kompositmaterials, die beim Mahlen des Komposits freigelegt werden, und verbessert so die Haftung zwischen Füllstoff und organischer Matrix. Als Haftvermittler sind die oben genannten Silane bevorzugt.

Die erfindungsgemäßen partikulären Kompositmaterialien eignen sich besonders als Füllstoffe für polymerisierbare Zusammensetzungen, d.h. Zusammensetzungen, die neben dem partikulären Kompositmaterial mindestens ein polymerisierbares Monomer und/oder Präpolymer und mindestens einen Polymerisationsinitiator enthalten. Der Anteil des partikulären Kompositmaterials liegt vorzugsweise im Bereich von 20 bis 90 Gew.-%, besonders bevorzugt 25 bis 70 Gew.-% und ganz besonders bevorzugt 30 bis 50 Gew.-%. Die Menge an organischem Bindemittel beträgt vorzugsweise 10 bis 80 Gew.-%, besonders bevorzugt 10 bis 30 Gew.-%, die Menge des Initiators 0,01 bis 5 Gew.-%, insbesondere 0,1 bis 1 Gew.-%.

Zur Herstellung polymerisierbarer Zusammensetzungen sind Kompositfüllstoffe mit einer Dichte von 1,5 bis 2,5 g/cm³, insbesondere 1,8 bis 2,2 g/cm³ bevorzugt. Die relativ geringe Dichte bedingt einen hohen Füllstoffvolumenanteil in der polymerisierbaren Zusammensetzung und bewirkt so eine zusätzliche Verringerung des Polymerisationsschrumpfes.

Neben dem Kompositfüller enthalten die polymerisierbaren Zusammensetzungen vorzugsweise zusätzlich weiteren partikulären anorganischen Füllstoff, insbesondere Quarz-, Glaskeramik-, Glaspulver oder Mischungen davon, besonders bevorzugt Glaspulver, ganz besonders bevorzugt Bariumglaspulver und/oder Strontiumglaspulver. Die mittlere Partikelgröße des Quarz-, Glaskeramik- und/oder Glaspulvers liegt vorzugsweise im Bereich von 0,4 bis 2 µm, besonders bevorzugt 0,4 bis 1,5 µm und ganz besonders bevorzugt 0,7 bis 1,0 µm. Der Anteil des Quarz-, Glaskeramik- und/oder Glaspulvers beträgt vorzugsweise 20 bis 70 Gew.-%, besonders bevorzugt 25 bis 50 Gew.-% und ganz besonders bevorzugt 30 bis 40 Gew.-% bezogen auf die Gesamtmasse der Zusammensetzung.

Die Zusammensetzungen können darüber hinaus eines der oben genannten gefällten Mischoxide und/oder einen der oben genannten Füllstoffe zur Erhöhung der Röntgenopazität enthalten, wie beispielsweise Ytterbiumtrifluorid. Das Mischoxid wird vorzugweise in einer Menge von 20 bis 70 Gew.-% eingesetzt, der Anteil des röntgenopaken Füllstoffs liegt vorzugsweise im Bereich von 1 bis 10 Gew.-%, besonders bevorzugt von 1 bis 5 Gew.-%. Die Partikelgröße des röntgenopaken Füllstoffs liegt vorzugsweise im Bereich von 100 bis 300 nm, insbesondere 180 bis 300 nm. Bevorzugte Mischoxide sind gefällte SiO₂/ZrO₂-Mischoxide, die vorzugsweise eine Partikelgröße von 200 bis 300 nm und insbesondere etwa 200 nm aufweisen.

Darüber hinaus können die Zusammensetzungen zur Einstellung des rheologischen Verhaltens ein organisch modifiziertes Schichtsilikat enthalten. Das Schichtsilikat wird bevorzugt in einer Menge von 0,05 bis 5 Gew.-%, besonders bevorzugt 0,1 bis 1 Gew.-% eingesetzt. Die Summe der Anteile des röntgenopaken Füllers und des Schichtsilikats beträgt vorzugsweise maximal 5 Gew.-%.

Die Gesamtmenge des zusätzlichen anorganischen Füllstoffs der polymerisierbaren Zusammensetzung liegt vorzugsweise im Bereich von 0,05 bis 85 Gew.-%, insbesondere 0,1 bis 56 Gew.-%. Der anorganische Füllstoff ist vorzugsweise mit einem Haftvermittler behandelt, also beispielsweise silanisiert.

Außerdem können die polymerisierbaren Zusammensetzungen übliche Additive und Zuschlagstoffe enthalten, vorzugsweise in einer Menge von 0,01 bis 2 Gew.-%.

Als organische Bindemittel, Polymerisationsinitiatoren, zusätzliche Füllstoffe und Additive eignen sich besonders die oben als Bestandteile des Kompositfüllers beschriebenen Substanzen. Bevorzugte Monomere zur Herstellung der polymerisierbaren Zusammensetzungen sind Benzylmethacrylat, ethoxyliertes Bisphenol-A-Dimethacrylat, Tetrahydrofurylmethacrylat und insbesondere Bisphenol-A-Dimethacrylat,ethoxyliertesBisphenol-A-Dimethacrylat gemäß der Formel (1) mit n = 1 und m = 2 und das Umsetzungsprodukt aus 2 mol Hydroxyethylmethacrylat (HEMA) und 1 mol Hexamethylendiisocyanat.

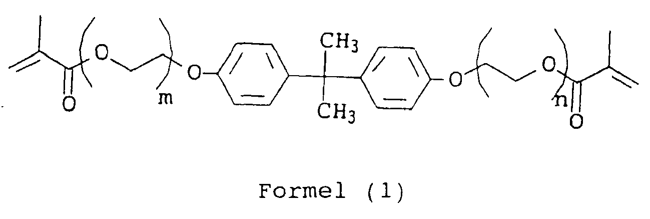

Als Additive kommen zusätzlich zu den oben genannten Materialien Beschleuniger, Farbstoffe, Pigmente, UV-Absorber, optische Aufheller und Gleitmittel in Betracht. Zusammensetzungen, die einen Photoinitiator enthalten, sind bevorzugt.

Die erfindungsgemäßen polymerisierbaren Zusammensetzungen setzen sich vorzugsweise wie folgt zusammen:
(i) 10 bis 80 Gew.-%, vorzugsweise 10 bis 30 Gew.-% organisches Bindemittel;
(ii) 0,01 bis 5 Gew.-%, vorzugsweise 0,1 bis 1 Gew.-% Polymerisationsinitiator;
(iii) 20 bis 90 Gew.-%, vorzugsweise 25 bis 75 Gew.-% Kompositfüller; und
(iv) ggf. 20 bis 70 Gew.-%, vorzugsweise 25 bis 50 Gew.-% Quarz-, Glaskeramik-, Glaspulver oder eine Mischung davon;
(v) ggf. 1 bis 10 Gew.-%, vorzugsweise 1 bis 5 Gew.-% röntgenopaker partikulärer Füllstoff;
(vi) ggf. 20 bis 70 Gew.-% gefälltes Mischoxid;
(vii) ggf. 0,05 bis 5 Gew.-%, vorzugsweise 0,1 bis 1 Gew.-% Schichtsilikat;
(viii) ggf. 0,01 bis 2 Gew.-% weitere Additive.

Die polymerisierbaren Zusammensetzungen eignen sich besonders als Dentalmaterialien. Unter dem Begriff Dentalmaterial werden Zahnfüllungsmaterialien, Materialien für Inlays oder Onlays, Zahnzemente, Verblendmaterialien für Kronen und Brücken, Materialien für künstliche Zähne oder sonstige Materialien für die prothetische, konservierende und präventive Zahnheilkunde verstanden.

Vorzugsweise dient der erfindungsgemäße Dentalwerkstoff als Zahnfüllungsmaterial. Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z.B. Benzoylperoxid und in die andere Paste z.B. N,N-Dimethyl-p-toluidin eingearbeitet. Durch Vermischen etwa gleicher Teile der beiden Pasten erhält man ein Zahnfüllungsmaterial, welches in wenigen Minuten aushärtet.

Wenn man bei den letztgenannten Materialien das Amin wegläßt und als Katalysator z.B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert. Das Inlay wird entnommen, bearbeitet und dann im Munde des Patienten in die Kavität einzementiert.

Die Erfindung bezieht sich nicht nur auf den Dentalwerkstoff, sondern auch auf daraus hergestellte Fertigteile, z.B. künstliche Zähne, Schalen, Inlays etc.

Nachfolgend wird die Erfindung anhand von Beispielen näher erläutert.

### Beispiel 1

### Herstellung eines partikulären Kompositmaterials (Kompositfüller)

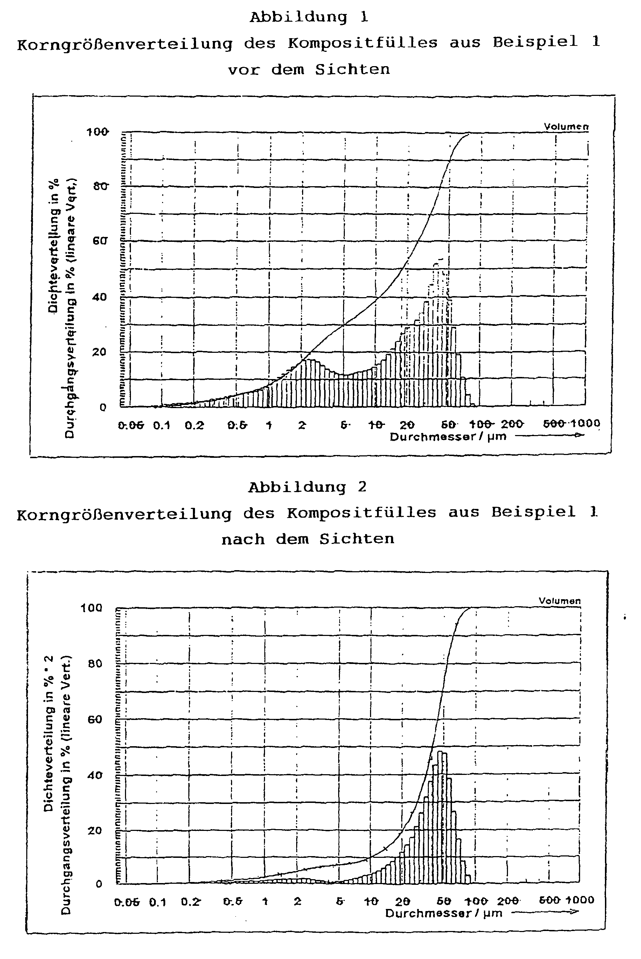

Zur Herstellung eines partikulären Kompositmaterials wurden die folgenden Komponenten in den angegebenen Mengen miteinander gemischt, und die Mischung bei 100 °C für 24 Stunden ausgehärtet, dann grob zerschlagen und anschließend in einer Kugelmühle gemahlen. Die mittlere Korngröße betrug 21 µm. Anschließend wurde der Feinanteil (< 10µm) und der Grobanteil (> 70 µm) mittels Sichter entfernt. Durch das Sichten verschiebt sich die mittlere Partikelgröße auf 37 µm. Die Korngrößenverteilung vor dem Sichten ist in Abbildung 1, die Korngrößenverteilung nach dem Sichten in Abbildung 2 gezeigt. Anschließend wurde die Hälfte des Materials mit 5 Gew.-% Methacryloxypropyltrimethoxysilan und 2 Gew.-% in Wasser silanisiert.

| Monomermischung zur Herstellung des Kompositfüllers: | |
|---|---|
| 1,10-Decandiol-dimethacrylat | 30 Gew.-% |
| Bisphenol-A-dimethacrylat | 39,95 Gew.-% |
| Urethandimethacrylat (UDMA) | 27 Gew.-% |
| Benzoylperoxid (50 %) | 3 Gew.-% |
| 2,6-Di-tert-butyl-para-kresol | 0,05 Gew.-% |

| Kompositfüller: | |
|---|---|
| Monomermischung | 20,5 Gew.-% |
| Bariumglaspulver (mittlere Partikelgröße 1,0 µm) | 54,5 Gew.-% |
| Ytterbiumfluorid | 25,0 Gew.-% |

### Beispiel 2

### Lichthärtendes Dentalmaterial (Komposit) auf der Basis von Kompositfüller

39,5 Gew.-% des partikulären Kompositmaterials gemäß Beispiel 1 wurden mit 39,5 Gew.-% Bariumglaspulver mit einer mittleren Teilchengröße von 1,5 µm, 15,5 Gew.-% einer Monomermischung mit der unten angegebenen Zusammensetzung und 2,5 Gew.-% einer Bentonepaste zu einer homogenen Paste gemischt. Die Bentonepaste setzt sich aus 12,5 Gew.-% Bentone (Schichtsilikat) und 87,5 Gew.-% der Monomermischung zusammen. Zur Bestimmung des Polymerisationsschrumpfes mittels Dilatometer wurden 0,1 g der Paste auf einem Glasplättchen fixiert, mit Quecksilber überschichtet und ein Wegaufnehmer schwimmend auf dem Quecksilber plaziert. Die Paste wurde durch das Glasplättchen hindurch mit einem Lichtpolymerisationsgerät (500 mW/cm²) für 60 Sekunden belichtet. Es wurde ein Polymerisationsschrumpf von 1,6 % gemessen.

| Monomermischung zur Herstellung des Dentalmaterials | |
|---|---|
| polymerisierbare Monomere: | |
| UDMA | 45 Gew.-% |
| Bisphenol-A-Dimethacrylat | 33,32 Gew.-% |
| Ethoxyliertes Bisphenol-A Dimethacrylat | 20 Gew.-% |

| Initiatormischung/Stabilisator: | |
|---|---|
| Campherchinon DL (Photoinitiator) | 0,33 Gew.-% |
| 4-Dimethylamino-benzoesäureethylester (Beschleuniger) | 0,6 Gew.-% |
| 2,4,6-Trimethylbenzoyl-diphenylphosphinoxid (Coinitiator) | 0,4 Gew.-% |
| 2,2,6,6-Tetramethylpiperidin-N-oxid | 0,012 Gew.-% |

| Additive: | |
|---|---|
| Blaues Fluoreszenzpigment (Lumilux Flu blau) | 0,04 Gew.-% |
| 2-(2'-Hydroxy-5'-methylphenyl)-benzotriazol (UV-Stabilisator) | 0,3 Gew.-% |

| Komposit | |
|---|---|
| Monomermischung | 15,5 Gew.-% |
| Bariumglaspulver (mittlere Partikelgröße 1,5 µm) | 39,5 Gew.-% |
| Kompositfüller | 39,5 Gew.-% |
| Schichtsilikatpaste (12,5 Gew.-% dispergiert in Monomermischung) | 2,5 Gew.-% |
| Ytterbiumtrifluorid | 3,0 Gew.-% |

### Beispiel 3

Es wurde analog zu Beispiel 2 ein lichthärtendes Dentalmaterial auf der Basis des Füllers gemäß Beispiel 1 hergestellt, der Füller jedoch nicht durch Sichten vom Fein- und Grobanteil befreit. Es wurde Füller der gleichen Charge verwendet. Der Polymerisationsschrumpf betrug 1,9 %.

## Patentansprüche

1. Zusammensetzung, die mindestens ein polymerisierbares Monomer und/oder Präpolymer, mindestens einen Polymerisationsinitiator und 20 bis 90 Gew.-%, bezogen auf die Gesamtmasse der Zusammensetzung, mindestens eines partikuläres Kompositmaterials enthält, **dadurch gekennzeichnet, daß** das partikuläres Kompositmaterial, eine mittlere Partikelgröße von 20 bis 50 µm aufweist und maximal 10 Gew.-%, bezogen auf die Masse des Kompositmaterials, Partikel mit einer Größe von < 10 µm enthält und daß die Zusammensetzung im wesentlichen frei von Füllstoff mit einer Partikelgröße von < 100 nm ist.

2. Zusammensetzung nach nach Anspruch 1, **dadurch gekennzeichnet, daß** das partikuläre Kompositmaterial eine maximale Partikelgröße von 70 µm aufweist.

3. Zusammensetzung nach Anspruch 1 oder 2, **dadurch gekennzeichnet, daß** das partikuläre Kompositmaterial durch Härten einer Mischung von
(a) 10 bis 80 Gew.-%, vorzugsweise 10 bis 30 Gew.-% organischem Bindemittel;
(b) 0,01 bis 5 Gew.-%, vorzugsweise 0,5 bis 2 Gew.-% Polymerisationsinitiator;
(c) 20 bis 90 Gew.-%, vorzugsweise 60 bis 88 Gew.-% anorganischem Füllstoff,
jeweils bezogen auf die Gesamtmasse des ungehärteten Kompositmaterials,
Mahlen der gehärteten Mischung und Klassieren des Mahlguts herstellbar ist.

4. Zusammensetzung nach Anspruch 3, **dadurch gekennzeichnet, daß** das partikuläre Kompositmaterial als Füllstoff Quarz-, Glaskeramik-, Glaspulver oder eine Mischung davon enthält.

5. Zusammensetzung nach Anspruch 4, **dadurch gekennzeichnet, daß** das partikuläre Kompositmaterial Glaspulver, vorzugsweise Bariumglaspulver und/oder Strontiumglaspulver enthält.

6. Zusammensetzung nach einem der Ansprüche 4 bis 5, **dadurch gekennzeichnet, daß** das Quarz-, Glaskeramik- und/oder Glaspulver eine mittlere Partikelgröße von 0,4 bis 1,5 µm, vorzugsweise 0,7 bis 1,0 µm aufweist.

7. Zusammensetzung nach einem der Ansprüche 3 bis 6, **dadurch gekennzeichnet, daß** das partikuläre Kompositmaterial 10 bis 50 Gew.-%, vorzugsweise 20 bis 30 Gew.-% röntgenopaken Füllstoff enthält.

8. Zusammensetzung nach Anspruch 7, **dadurch gekennzeichnet, daß** das partikuläre Kompositmaterial Ytterbiumfluorid enthält.

9. Zusammensetzung nach einem der Ansprüche 3 bis 8, **dadurch gekennzeichnet, daß** das partikuläre Kompositmaterial gefälltes Mischoxid enthält.

10. Zusammensetzung nach einem der Ansprüche 1 bis 9, **dadurch gekennzeichnet, daß** sie
(i) 10 bis 80 Gew.-% organisches Bindemittel;
(ii) 0,01 bis 5 Gew.-% Polymerisationsinitiator,
(iii) 20 bis 90 Gew.-% partikuläres Kompositmateria.l gemäß einem der Ansprüche 1 bis 9,
enthält, jeweils bezogen auf die Gesamtmasse der Zusammensetzung.

11. Zusammensetzung nach einem der Ansprüche 1 bis 10, **dadurch gekennzeichnet, daß** sie als weitere Komponente anorganischen Füllstoff enthält.

12. Zusammensetzung nach Anspruch 11, **dadurch gekennzeichnet, daß** sie als anorganischen Füllstoff Quarz-, Glaskeramik-, Glaspulver, oder eine Mischung davon enthält.

13. Zusammensetzung nach Anspruch 12, **dadurch gekennzeichnet, daß** sie Glaspulver, vorzugsweise Bariumglaspulver und/oder Strontiumglaspulver enthält.

14. Zusammensetzung nach Anspruch 12 oder 13, **dadurch gekennzeichnet, daß** das Quarz-, Glaskeramik- und/oder Glaspulver eine mittlere Partikelgröße von 0,4 bis 2 Am aufweist.

15. Zusammensetzung nach einem der Ansprüche 11 bis 14, **dadurch gekennzeichnet, daß** sie 25 bis 70 Gew.-%, vorzugsweise 30 bis 50 Gew.-% Quarz-, Glaskeramik- und/oder Glaspulver enthält.

16. Zusammensetzung nach einem der Ansprüche 11 bis 15, **dadurch gekennzeichnet, daß** sie als weitere Komponente röntgenopaken Füllstoff enthält.

17. Zusammensetzung nach Anspruch 16, **dadurch gekennzeichnet, daß** sie Ytterbiumfluorid enthält.

18. Zusammensetzung nach einem der Ansprüche 16 bis 17, **dadurch gekennzeichnet, daß** sie 1 bis 10 Gew.-% röntgenopaken Füllstoff enthält.

19. Zusammensetzung nach einem der Ansprüche 11 bis 18, **dadurch gekennzeichnet, daß** sie als weitere Komponente ein Schichtsilicat enthält.

20. Zusammensetzung nach Anspruch 19, **dadurch gekennzeichnet, daß** sie 0,05 bis 5 Gew.-% Schichtsilikat enthält.

21. Zusammensetzung nach einem der Ansprüce 1 bis 20, **dadurch gekennzeichnet, daß** sie zusätzlich gefälltes Mischoxid enthält.

22. Zusammensetzung nach Anspruch 21, **dadurch gekennzeichnet, daß** sie SiO₂/ZrO₂-Mischoxid enthalten.

23. Zusammensetzung nach einem der Ansprüche 21 bis 22, **dadurch gekennzeichnet, daß** das Mischoxid eine Partikelgröße von 200 bis 300 nm aufweist.

24. Zusammensetzung nach einem der Ansprüche 21 bis 23, **dadurch gekennzeichnet, daß** sie 20 bis 70 Gew.-% Mischoxid enthält.

25. Zusammensetzung nach einem der Ansprüche 1 bis 24, **dadurch gekennzeichnet, daß** sie zusätzlich 0,01 bis 2 Gew.-% Additive enthält.

26. Verwendung einer Zusammensetzung gemäß den Ansprüchen 1 bis 25 als Dentalmaterial, insbesondere als Zahnfüllungsmaterial, Material für Inlays oder Onlays, Zahnzement, Verblendmaterial für Kronen und Brücken, Material für künstliche Zähne.

## Claims

1. Composition that comprises at least one polymerisable monomer and/or prepolymer, at least one polymerisation initiator and 20 to 90 wt.%, based on the total weight of the composition, of at least one particulate composite material, **characterised in that** the particulate composite material has an average particle size of 20 to 50 µm and comprises maximum 10 wt.%, based on the weight of the composite material, of particles with a size < 10 µm and that the composition is essentially exempt of filler with a particle size of < 100 nm.

2. Composition according to Claim 1, **characterised in that** the particulate composite material has a maximum particle size of 70 µm.

3. Composition according to Claim 1 or 2, **characterised in that** the particulate composite material is manufactured by curing a mixture of
(a) 10 to 80 wt.%, preferably 10 to 30 wt.% organic binder;
(b) 0.01 to 5 wt.%, preferably 0.5 to 2 wt.% polymerisation initiator;
(c) 20 to 90 wt.%, preferably 60 to 88 wt.% inorganic filler,
each based on the total weight of the uncured composite material,
grinding the cured mixture and classifying the ground product.

4. Composition according to Claim 3, **characterised in that** the particulate composite material comprises quartz powder, glass ceramic powder, glass powder or a mixture thereof as the filler.

5. Composition according to Claim 4, **characterised in that** the particulate composite material comprises glass powder, preferably barium glass powder and/or strontium glass powder.

6. Composition according to one of Claims 4 to 5, **characterised in that** the quartz powder, glass ceramic powder and/or glass powder has an average particle size of 0.4 to 1.5 µm, preferably 0.7 to 1.0 µm.

7. Composition according to one of Claims 3 to 6, **characterised in that** the particulate composite material comprises 10 to 50 wt.%, preferably 20 to 30 wt.% of X-ray-opaque filler.

8. Composition according to Claim 7, **characterised in that** the particulate composite material comprises ytterbium fluoride.

9. Composition according to one of Claims 3 to 8, **characterised in that** the particulate composite material comprises precipitated mixed oxide.

10. Composition according to one of claims 1 to 9, **characterised in that** it comprises
(i) 10 to 80 wt.% organic binder;
(ii) 0.01 to 5 wt.% polymerisation initiator,
(iii) 20 to 90 wt.% particulate composite material according to one of Claims 1 to 9, each based on the total weight of the composition.

11. Composition according to one of Claims 1 to 10, **characterised in that** it additionally comprises an inorganic filler.

12. Composition according to Claim 11, **characterised in that** it comprises quartz powder, glass ceramic powder, glass powder or a mixture thereof as the inorganic filler.

13. Composition according to Claim 12, **characterised in that** it comprises glass powder, preferably barium glass powder and/or strontium glass powder.

14. Composition according to Claim 12 or 13, **characterised in that** the quartz powder, glass ceramic powder and/or glass powder has an average particle size of 0.4 to 2 µm.

15. Composition according to one of Claims 11 to 14, **characterised in that** it comprises 25 to 70 wt.%, preferably 30 to 50 wt.% of quartz powder, glass ceramic powder and/or glass powder.

16. Composition according to one of Claims 11 to 15, **characterised in that** it additionally comprises an X-ray-opaque filler.

17. Composition according to Claim 16, **characterised in that** it comprises ytterbium fluoride.

18. Composition according to one of Claims 16 to 17, **characterised in that** it comprises 1 to 10 wt.% of an X-ray-opaque filler.

19. Composition according to one of Claims 11 to 18, **characterised in that** it additionally comprises a layered silicate.

20. Composition according to Claim 19, **characterised in that** it comprises 0.05 to 5 wt.% layered silicate.

21. Composition according to one of Claims 1 to 20, **characterised in that** it additionally comprises a precipitated mixed oxide.

22. Composition according to Claim 21, **characterised in that** it comprises SiO₂/ZrO₂ mixed oxide.

23. Composition according to one of Claims 21 to 22, **characterised in that** the mixed oxide has a particle size of 200 to 300 nm.

24. Composition according to one of Claims 21 to 23, **characterised in that** it comprises 20 to 70 wt.% mixed oxide.

25. Composition according to one of Claims 1 to 24, **characterised in that** it additionally comprises 0.01 to 2 wt.% additives.

26. Use of a composition according to Claims 1 to 25 as a dental material, particularly as a tooth-filling material, material for inlays or onlays, tooth cement, facing material for crowns and bridges, material for false teeth.

## Revendications

1. Composition comprenant au moins un monomère polymérisable et/ou un prépolymère, au moins un initiateur de polymérisation et 20 à 90 % en poids, calculé sur la masse totale de la composition, d'au moins un matériau composite particulaire, **caractérisée en ce que** le matériau composite particulaire présente une taille moyenne de particule de 20 à 50 µm et contient au maximum 10 % en poids, calculé sur la masse du matériau composite, de particules ayant une taille < 10 µm et **en ce que** la composition est essentiellement exempte de charge ayant une taille de particule < 100 nm.

2. Composition selon la revendication 1, **caractérisée en ce que** le matériau composite particulaire présente une taille de particule maximale de 70 µm.

3. Composition selon la revendication 1 ou la revendication 2, **caractérisée en ce que** le matériau composite particulaire est susceptible d'être obtenu en faisant durcir un mélange de
(a) 10 à 80 % en poids, de préférence 10 à 30 % en poids d'un liant organique ;
(b) 0,01 à 5 % en poids, de préférence 0,5 à 2 % en poids d'un initiateur de polymérisation ;
(c) 20 à 90 % en poids, de préférence 60 à 88 % en poids d'une charge inorganique,
à chaque fois calculé sur la masse totale du matériau composite non durci,
en broyant le mélange durci et en effectuant un classement granulométrique par tamisage du broyat.

4. Composition selon la revendication 3, **caractérisée en ce que** le matériau composite particulaire contient, à titre de matière de charge, de la poudre de quartz, de la poudre de céramique vitreuse, de la poudre de verre, ou un mélange de celles-ci.

5. Composition selon la revendication 4, **caractérisée en ce que** le matériau composite particulaire contient de préférence de la poudre de verre de baryum et/ou de la poudre de verre de strontium.

6. Composition selon l'une quelconque des revendications 4 à 5, **caractérisée en ce que** la poudre de quartz, la poudre de céramique vitreuse et/ou la poudre de verre présentent une taille moyenne de particule de 0,4 à 1,5 µm, de préférence de 0,7 à 1,0 µm.

7. Composition selon l'une quelconque des revendications 3 à 6, **caractérisée en ce que** le matériau composite particulaire contient 10 à 50 % en poids, de préférence 20 à 30 % en poids d'une charge opaque aux rayons X.

8. Composition selon la revendication 7, **caractérisée en ce que** le matériau composite particulaire contient du fluorure d'ytterbium.

9. Composition selon l'une quelconque des revendications 3 à 8, **caractérisée en ce que** le matériau composite particulaire contient un oxyde mixte précipité.

10. Composition selon l'une quelconque des revendications 1 à 9, **caractérisée en ce qu'**elle contient
(i) 10 à 80 % en poids d'un liant organique ;
(ii) 0,01 à 5 % en poids d'un initiateur de polymérisation ;
(iii) 20 à 90 % en poids d'un matériau composite particulaire selon l'une des revendications 1 à 9,
à chaque fois calculé sur la masse totale de la composition.

11. Composition selon l'une quelconque des revendications 1 à 10, **caractérisée en ce qu'**elle contient comme composant supplémentaire une charge inorganique.

12. Composition selon la revendication 11, **caractérisée en ce qu'**elle contient comme charge inorganique de la poudre de quartz, de la poudre de céramique vitreuse et/ou de la poudre de verre, ou un mélange de celles-ci.

13. Composition selon la revendication 12, **caractérisée en ce qu'**elle contient de la poudre de verre, de préférence de la poudre de verre de baryum et/ou de la poudre de verre de strontium.

14. Composition selon la revendication 12 ou la revendication 13, **caractérisée en ce que** la poudre de quartz, la poudre de céramique vitreuse et/ou la poudre de verre présentent une taille moyenne de particule de 0,4 à 2 µm.

15. Composition selon l'une quelconque des revendications 11 à 14, **caractérisée en ce qu'**elle contient 25 à 70 %, de préférence 30 à 50 % en poids de poudre de quartz, de céramique vitreuse et/ou de verre.

16. Composition selon l'une quelconque des revendications 11 à 15, **caractérisée en ce qu'**elle contient comme composant supplémentaire une charge opaque aux rayons X.

17. Composition selon la revendication 16, **caractérisée en ce qu'**elle contient du fluorure d'ytterbium.

18. Composition selon l'une quelconque des revendications 16 à 17, **caractérisée en ce qu'**elle contient 1 à 10 % en poids d'une charge opaque aux rayons X.

19. Composition selon l'une quelconque des revendications 11 à 18, **caractérisée en ce qu'**elle contient comme composant supplémentaire un silicate lamellaire.

20. Composition selon la revendication 19, **caractérisée en ce qu'**elle contient 0,05 à 5 % en poids d'un silicate lamellaire.

21. Composition selon l'une quelconque des revendications 1 à 20, **caractérisée en ce qu'**elle contient en plus un oxyde mixte précipité.

22. Composition selon la revendication 21, **caractérisée en ce qu'**elle contient un oxyde mixte SiO₂/ZrO₂.

23. Composition selon l'une quelconque des revendications 21 à 22, **caractérisée en ce que** l'oxyde mixte présente une taille de particule de 200 à 300 nm.

24. Composition selon l'une quelconque des revendications 21 à 23, **caractérisée en ce qu'**elle contient 20 à 70 % en poids d'un oxyde mixte.

25. Composition selon l'une quelconque des revendications 1 à 24, **caractérisée en ce qu'**elle contient, de plus, 0,01 à 2 % en poids d'un additif.

26. Utilisation d'une composition selon l'une quelconque des revendications 1 à 25 comme matériau dentaire, en particulier comme matériau d'obturation dentaire, matériau pour incrustations en profondeur ou à recouvrement, ciment dentaire, facette prothétique pour couronnes et bridges, matériau pour dent artificielle.
